Europäisches Patentamt

European Patent Office

Office européen des brevets

(19)

(11) Numéro de publication: **0 090 687**
**A1**

(12) **DEMANDE DE BREVET EUROPEEN**

(21) Numéro de dépôt: 83400442.6

(51) Int. Cl.³: **A 61 M 25/00**

(22) Date de dépôt: **04.03.83**

(30) Priorité: **05.03.82 FR 8203674**

(71) Demandeur: **Durand, Alain Jean-Marie, Parc à Ballons Résidence Oméga, F-34000 Montpelier (FR)**

(43) Date de publication de la demande: **05.10.83**
**Bulletin 83/40**

(72) Inventeur: **Durand, Alain Jean-Marie, Parc à Ballons Résidence Oméga, F-34000 Montpelier (FR)**

(74) Mandataire: **Tony-Durand, Serge, Cabinet Tony-Durand 22, Boulevard Voltaire, F-75011 Paris (FR)**

(84) Etats contractants désignés: **BE CH DE GB IT LI NL SE**

(54) **Dispositif de cathétérisation, utilisable pour des perfusions dans les domaines médical et vétérinaire.**

(57) Dispositif de cathétérisation utilisable pour des perfusions dans les domaines médical et vétérinaire, comportant un cathéter à tubulure souple (3) et une aiguille (5) pour «tunneliser» la tubulure (3) sous la peau du patient.

L'aiguille (5) est fixée à la tubulure (3) par un dispositif empêchant tout rétrécissement de la lumière de la tubulure (3) à sa jonction avec l'aiguille (5) afin de permettre le libre coulissement d'un guide (2) nécessaire à la mise en place du cathéter dans le vaisseau, ce dispositif comprenant également une pince de sécurité (13) formée de deux branches (13a, 13b) articulées sur une charnière (26) et apte à serrer l'extrémité de la tubulure (3) sur la partie tubulaire rigide (8) d'un raccord (7).

Le cathéter selon l'invention peut être laissé en place dans un vaisseau pendant un temps atteignant plusieurs mois, et ce avec une sécurité accrue.

Dispositif de cathétérisation, utilisable pour des perfusions dans les domaines médical et vétérinaire.

La présente invention a pour objet un dispositif de cathétérisation, utilisable pour des perfusions dans les domaines médical et vétérinaire.

Plus particulièrement, ce dispositif comporte un cathéter à tubulure souple et des moyens pour "tunneliser" cette tubulure sous la peau du patient sur une distance déterminée entre un point de ponction d'un vaisseau sanguin et la surface externe de la peau du patient. Le cathéter comprend également un raccord de jonction de l'extrémité extérieure de la tubulure à une canalisation extérieure d'alimentation, tandis que les moyens de "tunnelisation" comprennent une aiguille solidaire de l'extrémité de la tubulure et destinée à être séparée de cette dernière après la tunnelisation pour permettre la fixation du racord à la tubulure.

Des cathéters pour perfusions par voie intravasculaire ou péritonéale, comportant une tubulure généralement souple introduite par la lumière d'une aiguille de ponction, sont bien connus, un cathéter de ce genre étant décrit par exemple dans le brevet français n° 1 064 445.

D'autres cathéters intravasculaires ont été réalisés dans le but de les mettre en place selon la technique décrite par SELDINGER dans la revue "Acta Radiologica". 39, 1953 PP 368-376. Cette technique consiste à ponctionner un vaisseau avec une aiguille, à introduire un guide dans la lumière de l'aiguille, à retirer l'aiguille en laissant le guide en place, puis à faire progresser un cathéter sur le guide, enfin à retirer le guide en laissant le cathéter en place.

Lorsque ces cathéters sont laissés en place sur un patient pendant un temps relativement court, ces dispositifs devenus classiques donnent généralement satisfaction.

Cependant, lorsqu'il est nécessaire de laisser de tels cathéters en place pendant un long délai, plusieurs semaines ou plusieurs mois, ou dans des conditions d'environnement particulières, on constate que ces cathéters risquent de permettre une contamination du patient par les germes ambiants qui vont emprunter le point de ponction initial pour atteindre le système vasculaire du patient. On constate également que de tels cathéters risquent de se désolidariser soit du patient, soit de la tubulure de perfusion, par exemple à l'occasion de mouvements que le patient peut faire inconsciemment.

Pour remédier aux risques de contamination du patient par la voie ouverte au point de ponction initial, une solution consiste à faire suivre au cathéter, entre le point de ponction du vaisseau et la surface externe du patient, non pas le chemin le plus court, mais un chemin de plusieurs centimètres, pratiquement parallèle à la peau du patient, à quelques millimètres sous la peau. Dans ces conditions, les germes extérieurs sont obligés de progresser dans une partie importante de chair peu vascularisée qui forme un obstacle naturel à leur progression. Cependant, la pratique de cette technique dénommée "tunnelisation" exige le recours à des pinces aux longs becs, ou à de grosses aiguilles, dans des conditions souvent traumatisantes soit pour le patient, soit pour la tubulure des cathéters.

D'autre part, les différents dispositifs utilisés jusqu'à présent pour rendre sûr le raccordement étanche de la tubulure externe, par exemple celle d'un perfuseur, avec le cathéter intra-vasculaire, ainsi que le positionnement du point de raccordement sur le patient, sont loin d'être parfaits et exigent couramment l'emploi de nombreux rubans adhésifs, points de suture, pansements, sans présenter la facilité d'emploi et la rapidité de pose demandées par les praticiens.

L'invention a pour but de remédier à ces inconvénients en réalisation un cathéter associé à des moyens pour "tunnéliser" la tubulure de celui-ci aisément, et pour raccorder ce cathéter en toute sécurité aux tubulures extérieures choisies. Les moyens de "tunnélisation" utilisée par l'invention, comprennent une aiguille solidaire d'une extrémité de la tubulure et destinée à être séparée de cette dernière après la tunnélisation pour permettre la fixation du raccord à la tubulure.

Cependant, compte tenu de l'importance des efforts d'arrachement subis par l'aiguille, lors de l'opération de tunnélisation, il convient que celle-ci soit très étroitement solidariée avec la tubulure correspondante, sans que cela entraîne un rétrécissement du diamètre intérieur de cette tubulure.

A ce sujet, il convient de noter que le brevet français 2 202 706 décrit un dispositif de fixation d'une aiguille sur une tubulure souple. Cette fixation est assurée par un sertissage de l'extrémité correspondante de cette aiguille sur l'extrémité respective de la tubulure correspondante après avoir disposé à l'intérieur de cette dernière une pièce pleine de renforcement en métal malléable. Ainsi le sertissage de l'extrémité de l'aiguille provoque un étranglement de cette pièce pleine de renforcement et la solidarisation de l'aiguille avec la tubulure résulte de la déformation imposée à cette tubulure du fait de la déformation et de l'étranglement de la pièce interne pleine. Mais une telle solution ne peut pas être envisagée pour le dispositif de cathétérisation selon l'invention. Il n'est également pas possible d'adopter la solution prévue dans le brevet français 1 125 688 qui concerne la fixation d'un embout creux sur un tuyau souple. En effet, les moyens de solidarisation existant dans ce cas seraient insuffisants pour résister aux efforts d'arrachement d'une aiguille servant à une opération de tunnélisation.

C'est pourquoi, conformément à l'invention, l'aiguille est fixée à la tubulure correspondante par un dispositif de fixation comportant un tube rigide, par exemple en acier inoxydable, dont le diamètre intérieur est égal à celui de la tubulure souple, encastré dans l'extrémité de cette tubulure, l'aiguille présentant, au regard de ce tube, un rétreint assurant la compression et le coincement de l'extrémité de cette tubulure contre le tube sans provoquer un rétrécissement du diamètre intérieur de la tubulure, ce qui assure une parfaite solidarisation de cette tubulure avec l'aiguille de tunnélisation.

D'autres particularités et avantages de l'invention apparaîtront au cours de la description qui va suivre, faite en référence aux dessins annexés sur lesquels on a représenté une forme de réalisation non limitative du dispositif de cathétérisation selon l'invention.

- La figure 1 est une vue de dessus en plan de l'ensemble des éléments constituant le dispositif de cathétérisation visé par l'invention.

- La figure 2 est une vue de dessus à l'échelle de l'aiguille de "tunnélisation".

- La figure 3 est une vue en coupe axiale à échelle agrandie suivant III-III de la figure 2, de la zone de jonction entre la tubulure et l'aiguille.

- La figure 4 est une vue en coupe transversales suivant IV-IV de la figure 3.

- La figure 5 est une vue de dessus en plan d'une pince ou "clamp" apte à serrer l'extrémité de la tubulure sur la partie tubulaire rigide du raccord du cathéter , sur lequel cette pince peut être montée après l'opération de tunnélisation.

- La figure 6 est une vue en élévation en bout de la pince de la figure 5 représentée ouverte.

- La figure 7 est une vue de dessus en plan du raccord du cathéter muni de la pince des figures 5 et 6, représentée ouverte mais avec la partie rigide du raccord engagée dans l'une des branches de la pince.

- La figure 8 est une vue analogue à la figure 7, montrant la pince ou "clamp" refermée sur la partie tubulaire rigide du raccord /et assurant sa solidarisation avec la tubulure souple enfilée sur cette partie tubulaire rigide.

- La figure 9 est une succession de schémas illustrant le procédé de mise en oeuvre du dispositif de cathétérisation selon l'invention.

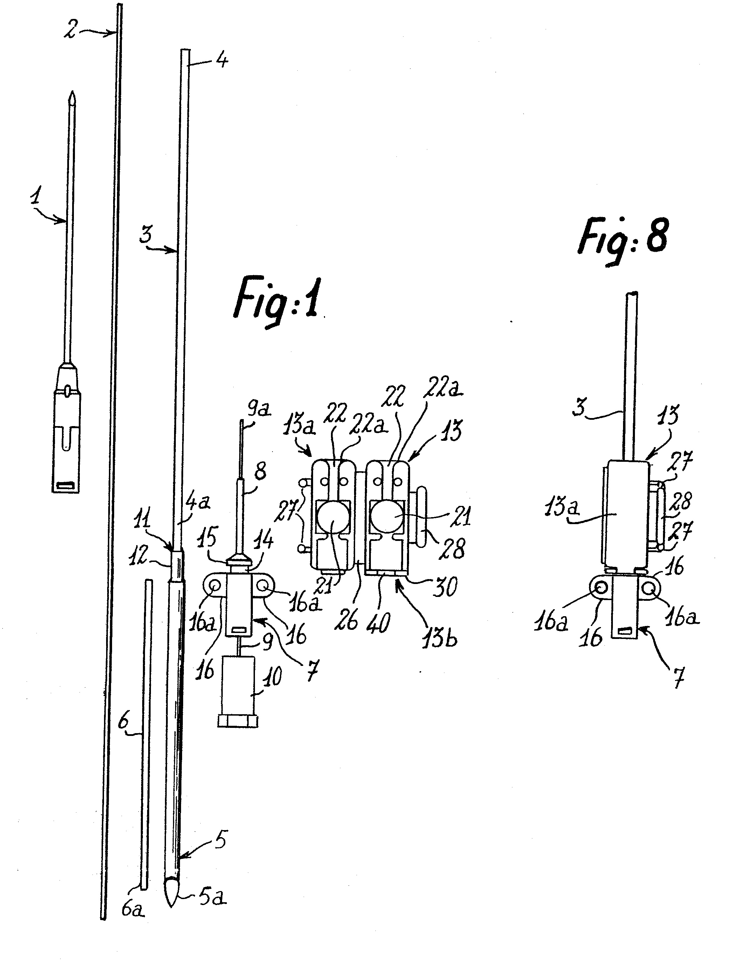

En se reportant à la figure 1, on voit l'ensemble des diverses pièces constitutives du dispositif de cathétérisation visé par l'invention, à savoir :

- une aiguille de ponction 1, un guide 2 constitué par un fil métallique destiné à être introduit dans l'aiguille 1

- une tubulure souple 3 du cathéter

- une aiguille de "tunnélisation" 5 fixée indissolublement à une extrémité de la tubulure souple 3

- un mandrin 6 destiné à obturer la lumière de l'aiguille 5

- un raccord conique normalisé 7 prolongé par un tube 8 dans la lumière duquel est placé un mandrin 9 muni d'un maneton 10 destiné à permettre une mise en place facile du raccord tubulaire 7, 8 dans l'extrémité de la tubulure souple 3

- Une pince ou "clamp" 13, destinée à parfaire la fixation de la tubulure 3 sur le raccord 7, 8 et à garantir cette tubulure contre des torsions ou des étirements.

Le cathéter proprement dit est constitué par l'assemblage de la tubulure souple 3 et du raccord 7, 8 après l'opération de tunnélisation.

L'ensemble des pièces constituant ce dispositif est destiné à l'exécution de perfusions au long cours, ou à des techniques de diagnostics cardio-vasculaires.

Pour permettre de procéder à ce type de cathétérisme, on peut choisir une tubulure souple 3 en polytétrafluoroéthylène, ou en polyuréthane, ou en élastomère de

silicone, ou en fluoroéthylène propylène, ou même en polyéthylène ou encore en tout autre matériau plastique approprié, conforme aux exigences de la biologie et de la législation médico-pharmaceutique.

A titre d'exemple numérique non limitatif, on
pourra décider d'utiliser pour un besoin précis de débit
de perfusion, ou de vitesse de prélèvement, une tubulure
3 en fluoroéthylène propylène de diamètre extérieur égal
à 1,67 mm et de diamètre intérieur de 1,07 mm.

Le guide 2 destiné à permettre l'introduction
du tube 3 peut avoir alors un diamètre de 0,89 mm, qui
permettra un glissement aisé du tube 3 devant coulisser
sur le guide 2.

L'extrémité 4 du tube 3 présente une finition
adéquate pour faciliter la pénétration de ce tube par le
point de ponction des tissus superficiels du vaisseau à
cathétériser.

D'autre part, du fait que le tube 3 muni de son
aiguille de tunnélisation 5 doit pouvoir coulisser sur
le guide 2, l'aiguille 5 doit être fixée sur la tubulure
3 sans provoquer de rétrécissement de la lumière de cette
dernière au point d'empêcher la pénétration et le glissement aisé du guide 2.

Ainsi, suivant une caractéristique essentielle
de l'invention, l'aiguille 5 est fixée à la tubulure 3
par un dispositif empêchant tout rétrécissement de la
lumière de la tubulure 3 à sa jonction avec l'aiguille 5.

Dans l'exemple de réalisation illustré à la
figure 3, pour assurer la jonction de la tubulure 3 à
l'aiguille 5 sans rétrécissement de la lumière de la tubulure, le dispositif de fixation de l'aiguille 5 à la tubulure 3 comporte un tube rigide 17 dont le diamètre intérieur d est égal à celui de la tubulure 3. L'extrémité 11
de la tubulure 3 se trouve ainsi encastrée entre le renfort annulaire interne rigide 17 et l'aiguille 5 qui présente, en regard du tube de renfort 17, un rétreint 12 qui solidarise l'aiguille 5 avec la tubulure 3 sans provoquer un rétrécissement du diamètre intérieur de cette dernière.

Cette solution est particulièrement appropriée pour solidariser une tubulure en PTFE ou en fluoroéthylène propylène, compte tenu des difficultés de collage de ces matériaux sur de l'acier inoxydable.

Pour compléter l'exemple numérique non limitatif donné ci-dessus, le diamètre d du tube 17 est égal à celui de la tubulure 3, donc est de 1,07 mm, son diamètre extérieur étant de 1,27 mm et sa longueur étant de 10 mm environ. Le rétreint 12 peut alors avoir un diamètre extérieur de 2,00 mm, alors que le corps cylindrique de l'aiguille 5 peut avoir un diamètre extérieur de 2,20 mm et une longueur de 5 à 15 mm environ.

Si l'on choisit d'utiliser un polyuréthane pour constituer la tubulure 3, cette opération de renfort intérieur, bien que préférable, peut éventuellement être remplacée par un collage approprié de l'aiguille 5, qui doit être fixée de façon sûre à la tubulure 3. L'essentiel est que la solidarisation de l'aiguille 5 et de la tubulure 3 doit être réalisée sans entraîner pour la lumière de cette dernière un rétrécissement tel que le guide 2 ne pourrait plus coulisser assez librement dans cette lumière.

En revenant à la figure 1, on voit sur celle-ci le mandrin 6 destiné à obturer l'aiguille 5, afin d'éviter un risque d'embolie gazeuse du patient lorsque la tubulure 3 a été mise en place dans un vaisseau, avant de procéder à la phase de pose appelée "tunnélisation".

Le mandrin 6 permet également d'éviter que le talon tranchant du biseau 5a de l'aiguille 5 puisse découper des morceaux de chair pendant la progression de cette aiguille 5 sous la peau du patient. Bien entendu, la longueur et le diamètre extérieur du mandrin obturateur 6 doivent être choisis en fonction de la position fixée pour l'extrémité 11 de la tubulure 3 dans la partie rétreinte 12 de l'aiguille 5, et des caractéristiques de cette dernière. D'une manière générale, la longueur du mandrin 6 doit être sensiblement égale à la distance existant entre l'extrémité 18 (figure 3) de la tubulure 3, située à l'intérieur de l'aiguille 5, et le talon 5a de cette même aiguille 5. On peut donner à ce mandrin 6 de préférence une section droite perpendiculaire à son axe pour l'extrémité destinée à venir s'appuyer sur la partie 18 de la tubulure 3.

Par contre, il est généralement préférable de prévoir une finition arrondie de l'extrémité 6a du mandrin 6 destinée à remplir l'extrémité biseauté 5a de l'aiguille 5.

Le mandrin 9, 9a logé dans la lumière du raccord 7, 8, le traverse entièrement, et son extrémité opposée à la partie tubulaire 8 se trouve fixée au maneton 10 (figure 1).

Le mandrin 9 est destiné à faciliter la mise en place du raccord tubulaire 7, 8 dans la lumière de l'extrémité de la tubulure 3 après coupe de celle-ci une fois l'opération de tunnélisation terminée. Le corps du raccord 7 est pourvu de façon connue, de deux oreilles latérales 16 percées chacune d'un trou 16a.

Suivant une caractéristique importante de l'invention, le raccord 7 est muni d'une pince ou "clamp" 13 (figures 1 et 5 à 7), apte à serrer l'extrémité 4a de la tubulure 3, opposée à l'extrémité 4 introduite dans le vaisseau, sur la partie tubulaire rigide 8 du raccord 7, et ce après exécution de la tunnélisation et séparation de l'aiguille 5 et de la tubulure 3 par coupe de l'extrémité de cette dernière. Le clamp 13 est agencé pour empêcher tout glissement relatif entre la partie tubulaire 8 et la tubulure 3, et pour être solidarisé fermement avec le raccord 7.

La pince ou clamp 13 est formée d'un boîtier en 2 parties 13a, 13b, articulées autour d'une charnière 26 formée par exemple d'un film souple en matière plastique. Ces deux parties peuvent être fermées l'une sur l'autre et la fermeture maintenue par exemple au moyen de deux ergots 27 formés latéralement sur la partie 13 a et qui viennent s'encliqueter sur les extrémités d'un rebord saillant 28 correspondant, s'étendant le long de la partie 13b.

La branche 13b de la pince 13 est munie, à son extrémité tournée vers le raccord 7, d'un redan 30 percé d'une ouverture 40 de passage pour le corps du raccord 7.

Le redan 30 est adapté pour venir s'introduire dans une gorge annulaire 14 ménagée dans la partie terminale du corps du raccord 7, du côté de la partie tubulaire 8, de telle sorte que la fermeture du boîtier constituant la pince 13 solidarise cette dernière de façon ferme avec le raccord 7.

Chaque partie 13a, 13b du boîtier est pourvue intérieurement d'une pastille 21 en un matériau élastique approprié, tel qu'un elastomère synthétique ou du caoutchouc, cette pastille étant logée respectivement dans un évidement 20 des branches 13a, 13b.

Des rainures complémentaires 22 sont ménagées dans chacune des extrémités des parties 13a, 13b, au-delà des pastilles 21, pour recevoir l'extrémité de la partie tubulaire rigide 8. Par ailleurs, de part et d'autre de l'une des rainures 22, par exemple celle qui est ménagée dans la branche 13b, une paire d'ergots 24 est agencée pour pouvoir venir s'encastrer dans des trous correspondants 25 formés sur les bords de la rainure 22 de l'autre branche 13a, à la fermeture du boîtier 13. Enfin, les rainures 22 présentent à leurs extrémités de sortie des évasements 22a afin d'éviter d'endommager la tubulure 3.

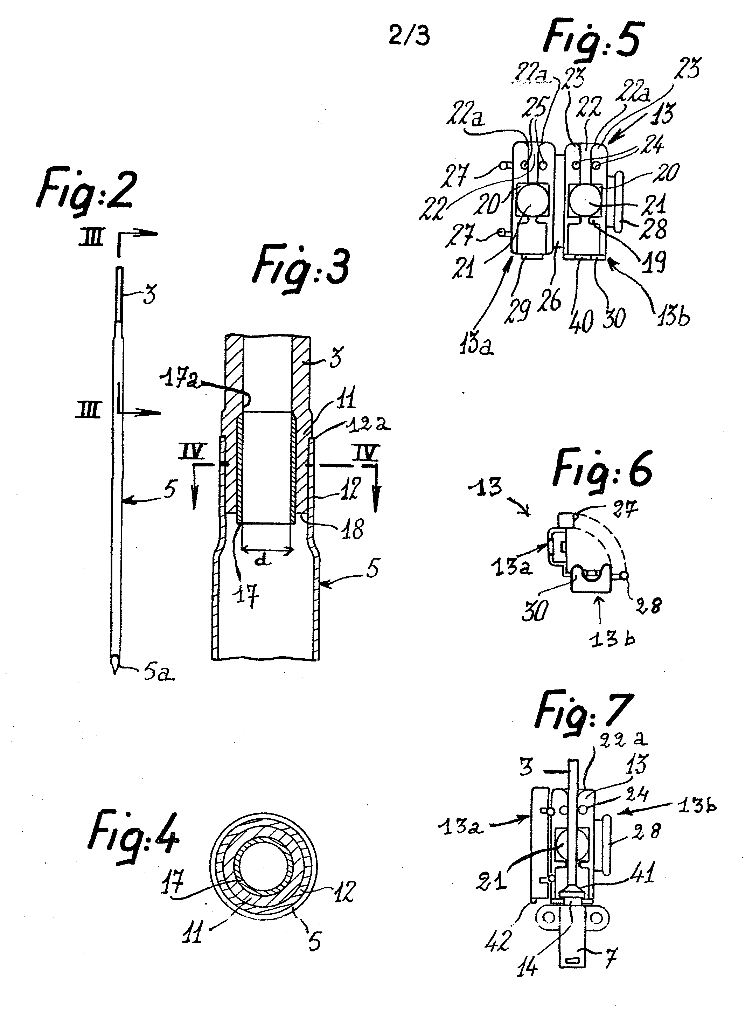

Lorsque le raccord 7 et sa partie rigide 8 sont introduits dans la branche 13b, la branche 13a étant ouverte, l'ensemble se présente comme illustré à la figure 7 : le redan 30 est engagé dans la partie étranglée constituée par la gorge 14, qui est reliée à la partie tubulaire 8 par une section conique 41, de sorte que le raccord 7 est bloqué en position contre la branche 13b. La partie tubulaire 8 prend appui sur la pastille 21 et s'étend dans la rainure 22. Lorsqu'on fait pivoter la branche 13a sur la charnière 26 pour la rabattre sur la branche 13b, les ergots 24 s'enfoncent dans les trous correspondants 25, une barrette saillante 42, formée à l'extrémité de la branche 13a en regard du redan 30 vient s'appliquer sur le raccord 7 de façon à contribuer au maintien en place de celui-ci, le boîtier constitué par la pince 13 se fermant par encliquetage élastique des ergots 27 sur les extrémités de la partie de la barrette 28.

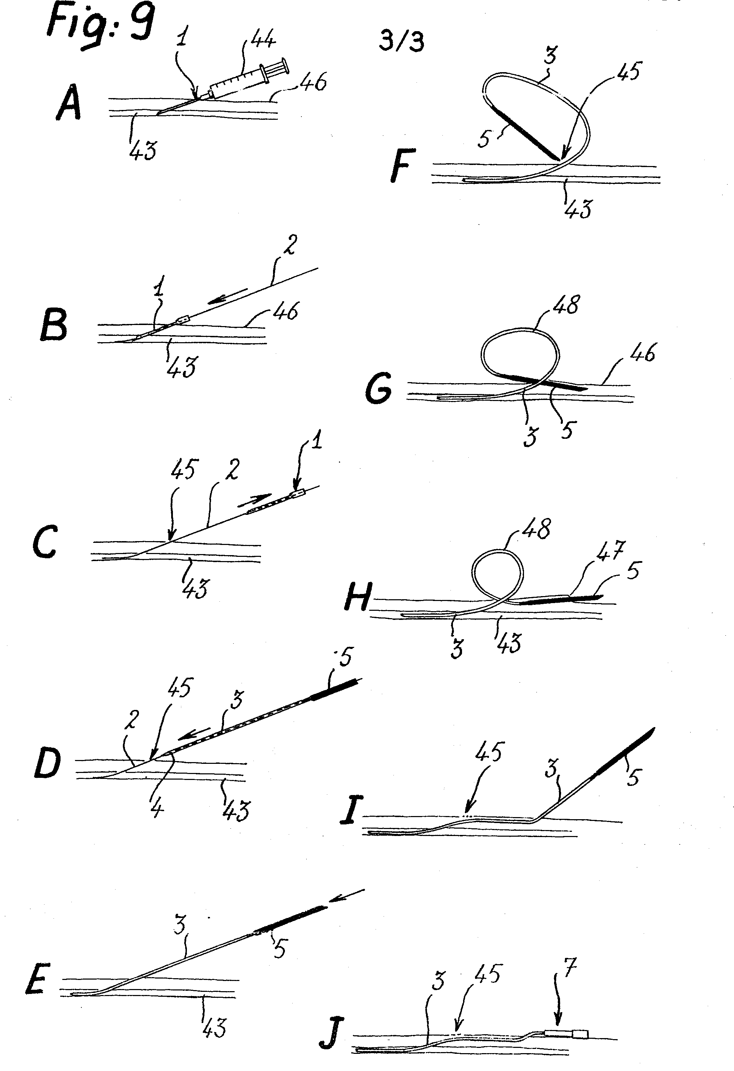

Au terme de cette manoeuvre, l'ensemble se présente sous la forme visible à la figure 8.

On décrira maintenant en référence aux schémas de la figure 9, le procédé de mise en oeuvre du dispositif de cathétérisation qui vient d'être décrit.

### Phase A

Après désinfection cutanée soigneuse, le praticien aborde le vaisseau 43 à cathéteriser, par exemple une veine sous-clavière ou une jugulaire interne, avec l'aiguille 1 dont la longueur peut être de 8 centimètres environ, montée sur une seringue 44 de 10 cm$^3$.

### Phases B et C

Un bon reflux de sang étant obtenu, le praticien retire la seringue 44 et introduit dans l'aiguille 1 le guide métallique spiralé 2, sur une quinzaine de centimètres. Le praticien retire ensuite l'aiguille 1 (Phase C) et la jette.

### Phase D

A l'aide d'un bistouri, le praticien agrandit légèrement l'orifice cutané 45 d'émergence du guide métallique 2 et introduit la tubulure 3 sur ce guide 2 en y enfonçant l'extrémité souple 4 dépourvue d'aiguille, l'extrémité opposée étant munie de l'aiguille 5. Le praticien positionne la tubulure 3 selon la méthode de Seldinger. Des repères sur la tubulure 3 indiquent au praticien la longueur de tubulure introduite.

### Phase E

Le praticien retire le guide métallique 2, puis introduit entièrement le mandrin obturateur 6 en matière plastique, d'une longueur de 7 cm environ, dans l'aiguille 5. Comme indiqué précédemment, le mandrin 6 évite les risques d'embolie gazeuse et empêche un possible découpage de morceaux de tissus du patient pendant l'opération de tunnélisation de la tubulure 3.

### Phase F

Le praticien saisit l'aiguille 5 et pique EXACTEMENT dans le point de ponction initial 45 en reclinant latéralement le cathéter.

### Phase G

Le praticien fait progresser l'aiguille 5 sous la peau 46, en la dirigeant vers le point d'émergence final 47 où elle doit ressortir. Cette phase opératoire est illustrée sur le schéma H.

### Phase I

Le praticien extrait de l'orifice d'émergence 47 l'aiguille 5 et tire sur celle-ci en entraînant doucement la tubulure 3 solidaire de l'aiguille, et jusqu'à disparition totale de la boucle 48 qui s'est formée au point de ponction initial 45.

### Phase J

Le praticien s'assure qu'il n'existe pas de pont cutané sous le cathéter au niveau du point initial de ponction 45, et qu'il n'existe pas non plus à ce niveau de la tubulure 3, une plicature pouvant gêner le débit des perfusions.

Le praticien coupe la tubulure 3 à environ 1 cm de l'aiguille sertie 5, et jette cette dernière. Il adapte le raccord femelle "luer-lock" 7, en introduisant à fond l'ensemble constitué par le raccord 7, 8 et le mandrin 9 dans la tubulure 3. Ensuite le praticien retire le mandrin 9 et son maneton 10 et le jette.

Le praticien ajoute alors la pince de sécurité 13 au cathéter comme représenté à la figure 7, en engageant le redan 30 dans la gorge 14 et la partie tubulaire 8 sur la pastille 21 et dans la gorge 22. La pince 13 est ensuite fermée par rabattement de la branche 13a avec introduction des ergots ou pions 24 dans les trous 25 et encliquetage des ergots 27 sur la barrette 28. Le praticien fixe alors à la peau du patient l'ensemble constitué par la pince 13 et le raccord femelle 7, soit en adaptant un ruban adhésif sur la pince, soit en passant un fil dans les trous 16a des oreilles 16, et ferme l'orifice initial de ponction 45 avec un fil de peau.

Le raccord 7 peut maintenant être branché sur la tubulure extérieure choisie.

Le cathéter et les moyens de tunnélisation qui viennent d'être décrits présentent deux avantages essentiels : d'une part la solidarisation de l'aiguille 5 avec la tubulure 3 est réalisée de façon à empêcher tout rétrécissement de la lumière de la tubulure 3 susceptible de gêner le coulissement du guide 2 ; d'autre part, la pince ou clamp 13 est agencée de telle manière qu'elle assure une parfaite solidarisation de la tubulure souple 3 sur la partie rigide 8 du raccord 7, empêchant tout glissement relatif entre ces pièces. Complémentairement, la pince 13 est fixée de manière très sûre au raccord 7, qui ne peut être désolidarisé de la pince que par une opération volontaire d'ouverture de celle-ci en dégageant les ergots 27 de la barrette de retenue 28. On remarquera que le tube de renfort 17 doit faire saillie par rapport à l'extrémité 18 de la tubulure 3 d'une distance minimale de 1 mm avant d'exécuter le rétreint 12 ; ceci évite un rétrécissement de la lumière de la tubulure 3 par l'extrémité 18.

De même, l'extrémité 12a du rétreint 12 doit se trouver en retrait par rapport à l'extrémité 17a du tube de renfort 17, pour la même raison.

Le cathéter selon l'invention permet d'exécuter aisément la tunnélisation et peut être raccordé en toute sécurité aux tubulures extérieures choisies.

L'invention n'est pas limitée à la forme de réalisation décrite et peut comporter de nombreuses variantes d'exécution.

Ainsi la pince 13 peut être réalisée de toute autre manière équivalente à celle décrite.

## REVENDICATIONS

1. Dispositif de cathétérisation, utilisable pour des perfusions dans les domaines médical et vétérinaire, comportant un cathéter à tubulure souple (3) et des moyens pour "tunnéliser" cette tubulure (3) sous la peau (46) du patient sur une distance déterminée entre un point de ponction (45) d'un vaisseau sanguin (43) et la surface externe de la peau (46) du patient, le cathéter comprenant également un raccord (7) de jonction à l'extrémité extérieure de la tubulure (3) à une canalisation extérieure d'alimentation, tandis que les moyens de "tunnélisation" comprennent une aiguille (5) solidaire d'une extrémité (4a) de la tubulure (3) et destinée à être séparée de cette dernière après la tunnélisation pour permettre la fixation du raccord (7) à la tubulure, caractérisé en ce que l'aiguille (5) est fixée à la tubulure (3) par un dispositif de fixation comportant un tube rigide (17), par exemple en acier inoxydable, dont le diamètre intérieur (d) est égal à celui de la tubulure souple (3), encastré dans l'extrémité de la tubulure (3), l'aiguille (5) présentant, au regard de ce tube (17), un rétreint (12) assurant la compression et le coincement de l'extrémité (11) de cette tubulure (3) contre le tube (17) sans provoquer un rétrécissement du diamètre intérieur (d) de la tubulure (3), ce qui assure une parfaite solidarisation de cette tubulure avec l'aiguille de tunnélisation (5).

2. Dispositif selon la revendication 1, dans lequel le raccord (7) du cathéter comporte un corps creux prolongé par une partie tubulaire rigide (8) adaptée pour pouvoir être introduite dans la tubulure souple (3), caractérisé en ce que le raccord (7) est muni d'une pince (13) apte à serrer l'extrémité (4a) de la tubulure (3) sur la partie rigide (8) afin d'empêcher tout glissement relatif entre ces deux pièces, et cette pince (13) est en outre agencée pour être solidarisée avec le raccord (7).

3. Dispositif selon la revendication 2, caractérisé en ce que la pince (13) est formée d'un boîtier en deux parties (13a, 13b), articulées autour d'une charnière (26) dont l'une présente, à l'une de ses extrêmités, un redan (30) percé d'une ouverture (40) de passage pour le corps du raccord (7), et qui est adapté pour venir s'introduire dans une gorge (14) ménagé dans ledit corps de telle sorte que la fermeture du boîter (13) solidarise le raccord (7) avec la pince (13).

4. Dispositif selon la revendication 3, caractérisé en ce que chaque partie (13a, 13b) du boîtier (13) est pourvue intérieurement d'une pastille (21) en un matériau élastique entre lesquelles la partie tubulaire (8) du raccord (7) est serrée lorsque le boîtier est fermé autour de celle-ci, des rainures complémentaires (22) étant ménagées dans les extrémités des parties (13a, 13b) du boîtier au-delà des pastilles (21) pour recevoir l'extrêmité de la partie tubulaire rigide (8), des ergots (24) pouvant en outre être prévus de part et d'autre de l'une de ces rainures (22) pour venir s'encastrer dans des trous complémentaires (25) de l'autre partie (13a) à la fermeture du boîtier (13).

5. Dispositif selon la revendication 4, caractérisé en ce que les rainures (22) sont évasées à leur extrémité de sortie afin d'éviter d'endommager la tubulure souple (3).

0090687

1/3

Fig:1

Fig:8

0090687

2/3

Fig.2  Fig.3  Fig.5  Fig.6  Fig.4  Fig.7

Fig. 9

0090687

3/3

# 0090687

## RAPPORT DE RECHERCHE EUROPEENNE

Office européen
des brevets

Numéro de la demande

EP 83 40 0442

### DOCUMENTS CONSIDERES COMME PERTINENTS

| Catégorie | Citation du document avec indication. en cas de besoin, des parties pertinentes | Revendication concernée | CLASSEMENT DE LA DEMANDE (Int. Cl. 3) |
|---|---|---|---|
| X,Y | FR-A-2 202 706 (H.T. DURAND) * Page 1, lignes 1-4, 28-39; page 2, lignes 1-17; page 3, lignes 13-32; revendications 1-4; figure 1 * | 1,2 | A 61 M 25/00 |
| Y | FR-A-1 125 688 (W. RÜSCH) * Page 1, colonne 1, lignes 1-41; colonne 2, lignes 23-40 * | 1,2 | |
| A | GB-A-1 447 237 (P. SADLER) * Page 1, lignes 22-40, 60-73, 90-98; page 2, lignes 1-45; figures 2,4 * | 2,3 | |
| A | GB-A-1 459 741 (P.I. PARKINSON) * Revendications 1,4-6,8,11 * | 2,4 | |
| A | FR-A-2 276 066 (ABBOTT) * Figure 1 * | 3,5 | DOMAINES TECHNIQUES RECHERCHES (Int. Cl. 3)<br><br>A 61 M |
| A | FR-A-2 012 505 (VIGGO AB) | | |
| A | GB-A-2 012 595 (J.L. PETERS et al.) | | |
| A | EP-A-0 001 106 (F. LEMIEUX) | | |
| | --- -/- | | |

Le présent rapport de recherche a été établi pour toutes les revendications

| Lieu de la recherche | Date d'achèvement de la recherche | Examinateur |
|---|---|---|
| LA HAYE | 20-06-1983 | ENGELBRECHT E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO Form 1503 03 82

**Office européen des brevets**

**RAPPORT DE RECHERCHE EUROPEENNE**

EP 83 40 0442

| | DOCUMENTS CONSIDERES COMME PERTINENTS | | Page 2 |

| Catégorie | Citation du document avec indication, en cas de besoin, des parties pertinentes | Revendication concernée | CLASSEMENT DE LA DEMANDE (Int. Cl. ³) |
|---|---|---|---|
| A | US-A-3 568 673 (C.C. COWLEY) | | |
| A | FR-A-2 263 789 (TRANSCODAN) | | |
| A | FR-A-2 121 965 (TECHNOLOGICAL SUPPLY) | | |
| A | US-A-3 574 306 (J.D. ALDEN) | | |

DOMAINES TECHNIQUES RECHERCHES (Int. Cl. ³)

Le présent rapport de recherche a été établi pour toutes les revendications

| Lieu de la recherche LA HAYE | Date d'achèvement de la recherche 20-06-1983 | Examinateur ENGELBRECHT E |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82